## Europäisches Patentamt

## European Patent Office

## Office européen des brevets

(19)

(11) Veröffentlichungsnummer: **0 003 763**
**B1**

(12) **EUROPÄISCHE PATENTSCHRIFT**

(45) Veröffentlichungstag der Patentschrift:
**17.02.82**

(51) Int. Cl.³: **A 61 B 17/18, A 61 F 1/00**

(21) Anmeldenummer: **79100326.2**

(22) Anmeldetag: **05.02.79**

(54) Osteosynthesehilfsmittel.

(30) Priorität: **16.02.78 DE 2806609**

(43) Veröffentlichungstag der Anmeldung:
**05.09.79 Patentblatt 79/18**

(45) Bekanntmachung des Hinweises auf die Patenterteilung:
**17.02.82 Patentblatt 82/7**

(84) Benannte Vertragsstaaten:
**BE CH FR GB IT NL SE**

(56) Entgegenhaltungen:
**DE-A-2 127 843**
**DE-A-2 154 272**
**DE-A-2 305 333**
**DE-A-2 320 373**
**DE-A-2 305 442**

(73) Patentinhaber: **Howmedica International, Inc. Zweigniederlassung Kiel, Professor-Küntscher-Strasse 1-5, D-2301 Schönkirchen üb. Kiel (DE)**

(72) Erfinder: **Härle, Anton, Dr., Schmeddingweg 75, D-4400 Münster (DE)**

(74) Vertreter: **Hauck, Hans, Dipl.-Ing. et al, Patentanwälte Dipl.-Ing.H.Hauck, Dipl.-Phys.W.Schmitz, Dipl.-Ing.E.Graalfs, Dipl.-Ing.W.Wehnert, Dipl.-Phys.W.Carstens Dr.-Ing.W.Döring Neuer Wall 41, D-2000 Hamburg 36 (DE)**

BUNDESDRUCKEREI BERLIN

### Osteosynthesehilfsmittel

Die Erfindung betrifft ein Osteosynthesehilfsmittel, wie Platte, Nagel, Schraube, aus einem eine entsprechende Festigkeit aufweisenden Werkstoff.

Derartige Osteosynthesehilfsmittel, insbesondere metallische Hilfsmittel, dienen der inneren Fixation einer Fraktur. Bei derartigen vollkommen gedeckten oder nicht vollkommen gedeckten Osteosynthesen stellt sich das Problem der Desinfektion in besonderer Weise, da es ein Bestreben sein muß, die Antibiotika nach Möglichkeit unmittelbar an den Ort des Infektionsgeschehens zu verbringen.

Aus einer Veröffentlichung (»Gentamycin-PMMA-Kette«, »Gentamycin-PMMA-Kugeln« der Firma E. Merck, Darmstadt 1977) ist eine mit einem Antibiotikum versehene Trägersubstanz bekanntgeworden, nämlich ein Polymethylmethacrylat, dem als Antibiotikum Gentamycin zugesetzt ist, wobei diese Trägermasse eine protrahierte Freisetzung des antibakteriellen Wirkstoffes in sicher bakterizider Konzentration gewährleistet und sicherstellt, daß trotz erfolgender Freisetzung des Antibiotikums die äußere Gestalt der Trägermasse erhalten bleibt.

Dieser bekannte Trägerstoff für das Antibiotikum wurde z. B. bei Knocheninfektionen eingesetzt, und zwar derart, daß auf einem chirurgischen Draht aufgezogene Kugeln in die ausgehöhlte Knochenmarkhöhle verbracht werden. Die konservative oder operative Behandlung offener und geschlossener Extremitätenfrakturen ist in einem bedeutsamen Prozentsatz (3—15%) durch das Auftreten von Knocheninfektionen gestört. Damit ist nicht nur die knöcherne Frakturkonsolidierung behindert oder stark verzögert, sondern häufig damit auch eine lokale Knochenzerstörung verbunden, die zu häufigen Operationen Anlaß geben kann. Neben der langen Behandlungsdauer sind meist kosmetisch unschöne Hautveränderungen und Störungen der normalen Körperproportionen nicht zu vermeiden und in Einzelfällen kann diese Gesundheitsstörung eine Amputation erfordern. Die Kombination von herkömmlichen Osteosynthesehilfsmitteln mit gleichzeitig eingelegten Gentamycin-PMMA-Ketten läßt keine ausreichend hohe Wirkstoffkonzentration an der Berührungsstelle von Metall und Knochen zu. Andererseits bedeutet das Raumerfordernis des Osteosynthesehilfsmittels schon für sich ein großes Problem bei der Weichteildeckung z. B. an Arm und Unterschenkel. Ein zusätzliches Einlegen von Ketten würde eine erhebliche Durchblutungsstörung des von der Knochenhaut ernährten Knochens bedeuten und die notwendige Weichteilbedeckung des Osteosynthesehilfsmittels kaum zulassen. Außerdem ist die Entfernung dieser zusätzlichen Ketten operativ aufwendig und schwierig.

In der DE-A-2 305 442 wird ein Implantat zur Befestigung im Inneren von Röhrenknochen beschrieben, wobei Zemente, Kunststoffe als Befestigungsmaterial im Knochen vermieden werden solien.

In dieser Literaturstelle wird vorgeschlagen, daß, damit nach dem ärztlichen Eingriff Entzündungen durch Krankheitserreger wirksam unterdrückt werden können, ein Pharmakadepot an geeigneter Stelle in dem Implantat installiert wird. In dieser Literaturstelle wird nicht ausgeführt, wie dieses Pharmakadepot ausgebildet sein soll, so daß davon ausgegangen werden kann, daß der Erfinder sich die Lehre gemäß der DE-A-2 154 272 zunutze macht, in welcher ein derartiges Pharmakadepot durch Einlagerung in Gelatine vorgeschlagen wird.

Aus der DE-A-2 127 843 ist ein metallisches Implantat bekanntgeworden, bei dem auf einem festen Metallsubstrat ein Überzug aus porösem Metall aufgebracht ist, das aus gleichem Werkstoff wie das feste Metallsubstrat und aus einer Vielzahl kleiner einzelner Teilchen besteht, die an den Berührungspunkten untereinander mit dem Substrat verschmolzen sind. Die poröse Schicht oder der poröse Überzug sollen das Einwachsen von Knochengewebe begünstigen. In dieser Druckschrift ist auch ausgeführt, daß die poröse Schicht mit einem Antibiotikum behandelt werden kann. In welcher Weise dies geschehen soll und das Antibiotikum nach dem Implantieren zur Wirkung kommt, ist nicht beschrieben.

Demgegenüber liegt der Erfindung die Aufgabe zugrunde, ein Osteosynthesehilfsmittel zu schaffen, das gleichzeitig als Träger für ein über lange Zeit wirkendes Antibiotikum benutzt wird, wobei auch nach längerer Zeit keine Oberflächenveränderung dieses Hilfsmittels vorliegt.

Diese der Erfindung zugrunde liegende Aufgabe wird dadurch gelöst, daß eine eine konstante Oberflächengestalt aufweisende, nicht resorbierbare und das Einwachsen von Gewebe unterbindende Trägermasse in zur Außenseite des Osteosynthesehilfsmittels offenen Aufnahmeräumen aufgenommen und fest angebracht ist, in der Antibiotika gleichmäßig verteilt untergebracht sind und die die Antibiotika kontrolliert freigibt.

Hiermit wird also ein Osteosynthesehilfsmittel geschaffen, das bei gleicher Festigkeit wie die zum Stand der Technik gehörenden Hilfsmittel nunmehr zusätzlich den Vorteil hat, daß bei seinem Anbringen gleichzeitig das Antibiotikum mit in den Wundbereich verbracht wird und daß bei Herauslösen des Hilfsmittels die Trägermasse wieder entfernt wird. Das Einwachsen von körpereigenem Gewebe in das Hilfsmittel ist ausgeschlossen, weil die Trägermasse die Oberflächengestalt nicht verändert und nicht resorbierbar ist.

Als Trägermasse kann ein geeigneter Knochenzement verwendet werden, wie er bei der Anbringung von Implantaten vielfach zum Einsatz gelangt, soweit er die protrahierte Freisetzung des Antibiotikums gewährleistet.

In den weiteren Unteransprüchen sind vorteilhafte Ausgestaltungen und Weiterentwicklungen dieses grundsätzlichen Gedankens erläutert, insbesondere soll darauf hingewiesen werden, daß es selbstverständlich möglich ist, die Oberfläche des Osteosynthesehilfsmittels und der in diesem oder an diesem gebildeten Aufnahmeräumen oder -flächen so zu bearbeiten, daß hierdurch die Haftfähigkeit zwischen Osteosynthesehilfsmittel und Trägermasse für das Antibiotikum verbessert wird.

Gemäß einem weiteren Merkmal der Erfindung wird darüber hinaus vorgeschlagen, daß die eigentliche Trägermasse auch an ihrer Außenseite aufgerauht sein kann, um damit eine bessere Fixation durch Knochenneubildung an der Oberfläche des Osteosynthesehilfsmittels zu gewährleisten.

Bei einer weiteren Ausführungsform der erfindungsgemäßen Überlegung wird so vorgegangen, daß der Bereich des Osteosynthesehilfsmittels, der gegebenenfalls gebogen werden muß, von vornherein von Trägermasse freibleibt, so daß ein problemloses Biegen des Hilfsmittels möglich ist. Anschließend kann dann dieser Bereich ebenso wie die beispielsweise verwendeten Schrauben oder dergleichen mit zusätzlichen Trägermassen, Plättchen oder Hütchen abgedeckt werden.

Zusammenfassend ist festzustellen, daß durch den erfindungsgemäßen Vorschlag auf jeden Fall eine außerordentlich hohe Wirkstofffreisetzung im unmittelbaren Bereich der Fraktur oder des operierten Bereiches gewährleistet ist.

In der beigefügten Zeichnung ist ein Ausführungsbeispiel der Erfindung dargestellt. Die Zeichnung zeigt dabei in

Fig. 1 eine schaubildliche Darstellung einer leicht gewölbten Knochenplatte, wie sie heute zum Stand der Technik gehört, in

Fig. 2 eine entsprechend einem Vorschlag gemäß der Erfindung bearbeitete, abgeänderte Ausführungsform gemäß Fig. 1, in

Fig. 3 das Osteosynthesehilfsmittel gemäß Fig. 2 mit angebrachtem Antibiotikum, in

Fig. 4 einen Schnitt gemäß der Linie IV-IV in Fig. 1, in

Fig. 5 einen Schnitt gemäß der Linie V-V in Fig. 2 und in

Fig. 6 einen Schnitt gemäß der Linie VI-VI in Fig. 3.

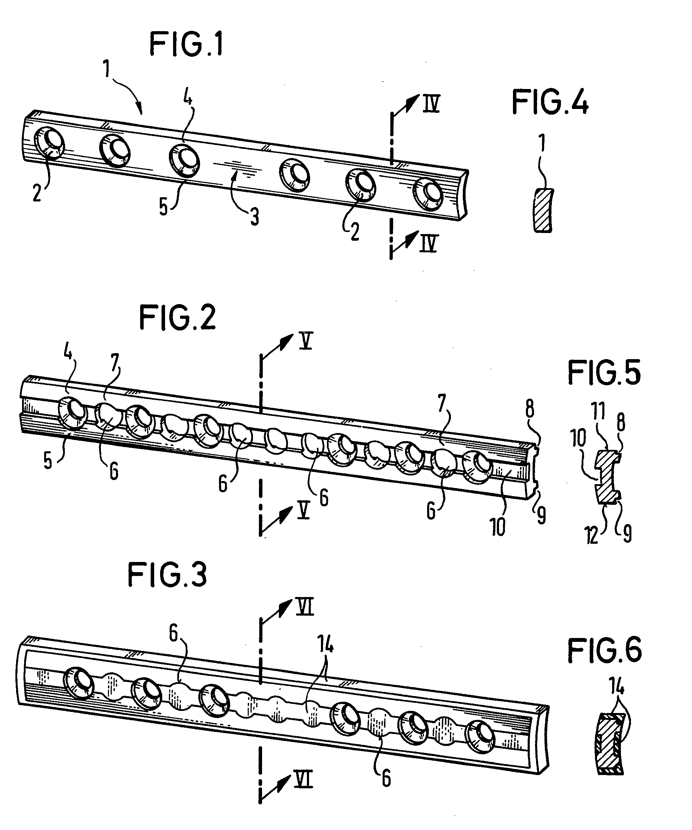

In Fig. 1 und 4 ist eine leicht gewölbte Knochenplatte dargestellt, die allgemein mit 1 bezeichnet ist und bei welcher Räume 2 für die der Festlegung der Platte am Knochen oder an der gegenüberliegenden Platte dienenden Schrauben vorgesehen sind.

Im mittleren Bereich der Platte 1 ist bei dem dargestellten Ausführungsbeispiel gemäß Fig. 1 ein schraubenloser Bereich 3 vorgesehen, der die Fraktur überbrückt.

Es ist erkennbar, daß die durch diese Platte 1 erzielte Zug- und Biegefestigkeit durch die Bereiche 4 bzw. 5 gebildet werden, die sich seitlich neben der Bohrung 2 zur Aufnahme einer Schraube befinden, da hier die kleinsten Materialbereiche vorliegen.

Von dieser Überlegung ausgehend wird nunmehr gemäß der Erfindung vorgeschlagen, den Rohling einer in Fig. 1 dargestellten Platte 1 entsprechend der Darstellung in Fig. 2 zu bearbeiten, d. h., es werden zusätzliche, als Aufnahmeräume dienende Bohrungen 6 angebracht, durch die aber die Biegefestigkeit der Platte nicht beeinträchtigt wird, da diese Bohrungen einen in Fig. 2 mit 7 bezeichneten Rand zur Außenseite hin stehenlassen, der wenigstens die gleiche Festigkeit aufweist wie die Bereiche 4 bzw. 5.

Außerdem kann diese Platte, wie in Fig. 5 aus der Schnittdarstellung gemäß der Linie V-V ersichtlich ist, mit zusätzlichen Ausfräsungen 8 und 9 bzw. 10 versehen sein. Diese Ausfräsungen 8 bis 10 dienen ebenso wie die Bohrungen 6 der Aufnahme einer das Antibiotikum tragenden Trägermasse 14.

Schließlich ist in Fig. 3 das Osteosynthesehilfsmittel gemäß Fig. 2 — nunmehr aber ausgerüstet mit der Trägermasse 14 — dargestellt, und es ist erkennbar, daß diese Trägermasse 14 nicht nur in den vorgenannten Ausnehmungen 8 bis 10 bzw. der Bohrung 6 angeordnet ist, sondern zusätzlich auch an den Außenkanten 11 und 12 angelagert sein kann, so daß damit die Möglichkeit geschaffen wird, eine hohe Menge an Trägermasse 14 mit dem eigentlichen, in den dargestellten Ausführungsbeispielen metallisch ausgebildeten Osteosynthesehilfsmittel zu verbinden, womit gleichzeitig eine relativ große Antibiotikamenge an den Infektionsherd gebracht werden kann. Durch die protrahierte Freisetzung dieses Antibiotikums ist nunmehr eine Langzeitbehandlung möglich, wobei gleichzeitig aber durch das Freisetzen des Antibiotikums keine räumliche Veränderung des Osteosynthesehilfsmittels erfolgt.

Gleichzeitig wird bei Entfernen des Osteosynthesehilfsmittels auch die Trägermasse mit entfernt, so daß in einer einzigen Operation sowohl das Antibiotikum wie auch das Osteosynthesehilfsmittel entfernt werden können.

Während bei dem in Fig. 1 dargestellten Ausführungsbeispiel ein lochloser oder schraubenloser Bereich 3 vorgesehen ist, ist bei der erfindungsgemäßen Anordnung gemäß Fig. 2 und 3 dieser Bereich ebenfalls mit Aufnahmeräumen 6 versehen, der der Aufnahme der Trägermasse dienen soll.

In der Zeichnung nicht dargestellt ist die Möglichkeit, die Trägermasse an ihrer Außenseite aufzurauhen, wobei durch diese Aufrauhung eine wesentlich bessere Fixation durch Knochenneubildung an der Oberfläche des Hilfsmittels gewährleistet wird, da die kritische Grenzfläche Metall/Knochenspangiosa umgangen wird. Bei diesem letztgenannten Vorschlag wird also, kombiniert mit einer hohen Wirkstofffreisetzung, im entscheidenden Bereich gleichzeitig ein gutes Einwachsen und eine gute Verträglichkeit des eingepflanzten Hilfsmittels erreicht.

## Patentansprüche

1. Osteosynthesehilfsmittel, wie Platte, Nagel, Schraube, aus einem eine entsprechende Festigkeit aufweisenden Werkstoff, dadurch gekennzeichnet, daß eine konstante Oberflächengestalt aufweisende, nicht resorbierbare und das Einwachsen von Gewebe unterbindende Trägermasse (14) in zur Außenseite des Osteosynthesehilfsmittels offenen Aufnahmeräumen (6, 8, 9, 10) aufgenommen und fest angebracht ist, in der Antibiotika gleichmäßig verteilt untergebracht sind und die die Antibiotika kontrolliert freigibt.

2. Osteosynthesehilfsmittel nach Anspruch 1, dadurch gekennzeichnet, daß die Aufnahmeräume (6, 8, 9, 10) mit einer die Haftfähigkeit der Trägermasse an dem Osteosynthesehilfsmittel erhöhenden Oberflächengestaltung versehen sind.

3. Osteosynthesehilfsmittel nach Anspruch 1 oder 2, dadurch gekennzeichnet, daß die Außenseite der Trägermasse (14) aufgerauht ist.

4. Osteosynthesehilfsmittel nach einem der Ansprüche 1 bis 3, gekennzeichnet durch einen trägermassenfreien Bereich des Osteosynthesehilfsmittels zur Durchführung von Biegungen.

5. Osteosynthesehilfsmittel nach Anspruch 4, gekennzeichnet durch trägermassenfreie Bereiche nachträglich abdeckende Häubchen oder dergleichen.

## Claims

1. An osteosynthesis aid, such as a plate, nail, screw, of a material having a corresponding strength, characterized in that a carrier material (14) having a consistent surface configuration and being incapable of being resorbed while preventing the ingrowth of tissue is accommodated and fixedly arranged in reception spaces (6, 8, 9, 10) opening towards the outside of the osteosynthesis aid, in which antibiotica are accommodated in uniform distribution and which dispenses the antibiotica in a controlled manner.

2. An osteosynthesis aid according to claim 1, characterized in that the reception spaces (6, 8, 9, 10) are provided with a surface configuration enhancing the adhesion of the carrier material to the osteosynthesis aid.

3. An osteosynthesis aid according to any one of the claims 1 to 3, characterized in that the outer surface of the carrier material (14) is roughened.

4. An osteosynthesis aid according to any one of the claims 1 to 3, characterized by a region of the osteosynthesis aid which is free of the carrier material in order to perform bending movements.

5. An osteosynthesis aid according to claim 4, characterized by small caps or the like afterwards covering the regions free of carrier material.

## Revendications

1. Moyen auxiliaire d'ostéosynthèse, tel qu'attelle, clou, vis, en un matériau présentant une résistance appropriée, caractérisé par le fait qu'une masse de support (14), présentant une forme de surface constante, non résorbable et entravant le bourgeonnement du tissu, est logée et disposée de façon fixe dans des cavités réceptrices (6, 8, 9, 10), ouvertes vers le côte extérieur du moyen auxiliaire d'ostéosynthèse, des antibiotiques étant introduits dans cette masse à l'état uniformément distribué, et ladite masse libérant les antibiotiques de façon contrôlée.

2. Moyen auxiliaire d'ostéosynthèse selon la revendication 1, caractérisé par le fait que les cavités réceptrices (6, 8, 9, 10) sont munies d'une conformation de surface qui augmente l'adhérence de la masse de support au moyen auxiliaire d'ostéosynthèse.

3. Moyen auxiliaire d'ostéosynthèse selon la revendication 1 ou 2, caractérisé par le fait que le côte extérieur de la masse de support (14) est rugueuse.

4. Moyen auxiliaire d'ostéosynthèse selon l'une des revendications 1 à 3, caractérisé par une zone du moyen auxiliaire d'ostéosynthèse, qui est dépourvue de masse de support, pour l'exécution de coudes.

5. Moyen auxiliaire d'ostéosynthèse selon l'une quelconque des revendications 1 à 4, caractérisé par des calottes ou des éléments similaires, qui recouvrent après coup des zones dépourvues de masse de support.

FIG.1

FIG.4

FIG.2

FIG.5

FIG.3

FIG.6